# EUROPEAN PATENT APPLICATION

(11) **EP 4 378 967 A1**
(43) Date of publication of application: **05.06.2024**
(21) Application number: 22869862.7
(22) Date of filing: 06.09.2022
(51) Int. Cl.: C08F 20/56, A61K 6/30, A61K 6/60, A61K 6/887, C07C 233/38, C08L 33/26

(54) **(METH)ACRYLAMIDE COMPOUND, MONOMER COMPOSITION, COMPOSITION FOR DENTAL MATERIAL, AND DENTAL MATERIAL**

(30) Priority: 17.09.2021 JP 2021152359; 17.09.2021 JP 2021152360
(71) Applicant: Mitsui Chemicals, Inc., Tokyo 105-7122 (JP)
(72) Inventor: KAKINUMA, Naoyuki, Sodegaura-shi, Chiba 299-0265 (JP)
(74) Representative: Hoffmann Eitle
(86) International application number: PCT/JP2022/033483
(87) International publication number: WO 2023/042715

(57) **Abstract**

A monomer composition for a dental material containing a (meth)acrylamide compound (A) including a cyclic structure and a (meth)acrylamide group.

## Description

### Technical Field

The present disclosure relates to a (meth)acrylamide compound, a monomer composition, a composition for a dental material, and a dental material.

### Background Art

A (meth)acrylate compound is widely used as a monomer in a curable composition.

For example, Patent Literature 1 discloses a (meth)acrylate (D) as a monomer which can provide a cured product having both high toughness and rigidity. The (meth)acrylate (D) in this literature is a reaction product of a thiol compound (A) having two or more mercapto groups, an iso(thio)cyanate compound (B) having two or more iso(thio)cyanate groups, and a hydroxy (meth)acrylate compound (C) having one or more polymerizable groups. This literature also discloses a composition containing the (meth)acrylate (D).

Patent Literature 1: WO 2019/107323 A1

### SUMMARY OF INVENTION

### Technical Problem

The (meth)acrylate compound may be used as a monomer contained in a monomer composition for a dental material. The monomer composition for a dental material may be used as a dental repair agent such as a composite resin.

Since the monomer composition for a dental material is used in the oral cavity with high humidity, the resulting cured product tends to absorb water. In a case in which the water absorption rate of the cured product is high, mechanical properties may be deteriorated.

Therefore, it is required to suppress an increase in water absorption rate of the cured product.

The (meth)acrylate compound is known as a monomer exhibiting polymerizability. For example, the (meth)acrylate compound may be used as a monomer contained in a monomer composition for a dental material.

The (meth)acrylate compound is a monomer used for various applications, and it is required to study a compound having a novel structure which has not been known heretofore.

An object of Embodiment A of the disclosure is to provide a (meth)acrylamide compound with which a cured product can be obtained in which an increase in water absorption rate is suppressed, a monomer composition containing the (meth)acrylamide compound, a composition for a dental material, and a dental material.

An object of Embodiment A-1 of the disclosure is to provide a (meth)acrylamide compound having a novel structure, a monomer composition containing the (meth)acrylamide compound, a composition for a dental material, and a dental material.

### Solution to Problem

Means for solving the above problems include the following aspects.

Note that an aspect of Embodiment A of the disclosure includes the following <1> to <15>. Among them, the following <6> to <15> are also included in an aspect of Embodiment A-1 of the disclosure.
<1> A (meth)acrylamide compound (A) including a cyclic structure and a (meth)acrylamide group.
<2> The (meth)acrylamide compound (A) according to <1>, in which the (meth)acrylamide compound (A) is a reaction product of a (meth)acrylic compound (X), and a primary amine compound (Y) including two amino groups and a cyclic structure, the (meth)acrylic compound (X) being at least one selected from the group consisting of a (meth)acrylic acid and a halide of a (meth)acrylic acid,.
<3> The (meth)acrylamide compound (A) according to<2>, in which the primary amine compound (Y) includes a compound represented by any one of the following Formulas (1-1) to (1-7). 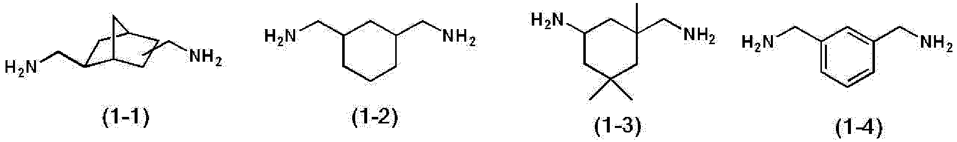 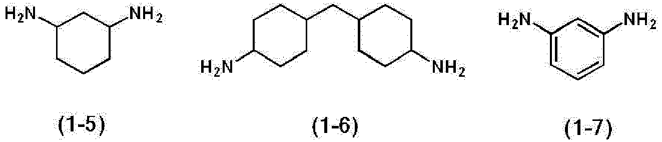
<4> The (meth)acrylamide compound (A) according to any one of <1> to <3>, which is a compound represented by the following Formula (1). 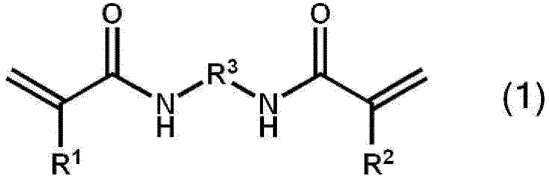 In Formula (1), each of R¹ and R² independently represents a hydrogen atom or a methyl group, and R³ represents a divalent group including a cyclic structure.
<5> The (meth)acrylamide compound (A) according to <4>, in which the R³ in Formula (1) is a group represented by the following Formula (1a). 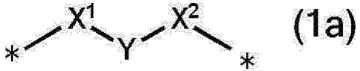 In Formula (1a), each of X¹ and X² h independently represents a single bond or a methylene group, Y represents a divalent linking group having from 6 to 13 carbon atoms including an alicyclic structure or an aromatic structure, and each of two *'s represents a bonding position.
<6> The (meth)acrylamide compound (A) according to <1>, containing two (meth)acrylamide groups and a divalent alicyclic hydrocarbon group,
   in which a nitrogen atom in each of the two (meth)acrylamide groups isbonded to the divalent alicyclic hydrocarbon group via a methylene group.
<7> The (meth)acrylamide compound (A) according to <6>, which is a reaction product of:
   a (meth)acrylic compound (X) which is at least one selected from the group consisting of a (meth)acrylic acid and a halide of a (meth)acrylic acid, and
   a primary amine compound (Y1) that includes two amino groups and the divalent alicyclic hydrocarbon group, in which a nitrogen atom in each of the two amino groups isbonded to the divalent alicyclic hydrocarbon group via a methylene group.
<8> The (meth)acrylamide compound (A) according to <7>, in which the primary amine compound (Y1) includes a compound represented by the following Formula (1-1) or (1-2). 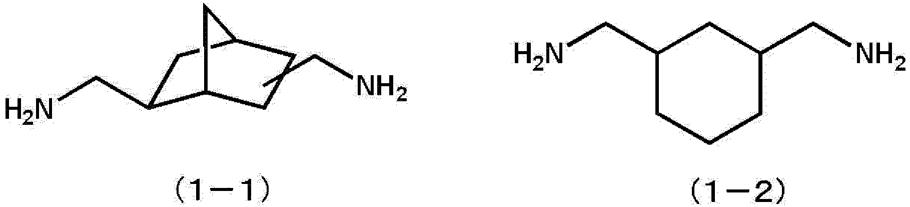
<9> The (meth)acrylamide compound (A) according to any one of <6> to <8>, which is a compound represented by the following Formula (1A). 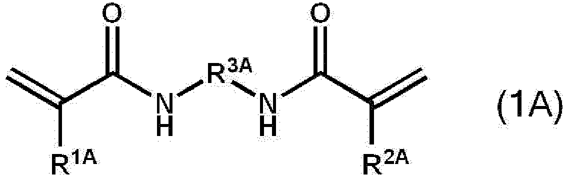 In Formula (1A), each of R^{1A} and R^{2A} independently represents a hydrogen atom or a methyl group, and R^{3A} is a divalent group formed by linking a methylene group, a divalent alicyclic hydrocarbon group, and a methylene group in this order.
<10> The (meth)acrylamide compound (A) according to <9>, in which the R^{3A} in Formula (1A) is a group represented by the following Formula (1Aa). 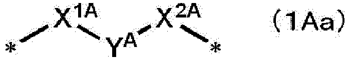 In Formula (1Aa), each of X^{1A} and X^{2A} independently represents a methylene group, Y^{A} is a divalent alicyclic hydrocarbon group having from 6 to 9 carbon atoms, and each of two *'s represents a bonding position.
<11> The (meth)acrylamide compound (A) according to any one of <1> to <10>, in which a molecular weight is from 150 to 500.
<12> A monomer composition, containing the (meth)acrylamide compound (A) according to any one of <1> to <11>.
<13> The monomer composition according to <12>, which is used for a dental material.
<14> A composition for a dental material, containing the monomer composition according to <12> or <13> and a polymerization initiator.
<15> A dental material, containing a cured product of the composition for a dental material according to <14>.

### Advantageous Effects of Invention

According to Embodiment A of the disclosure, it is possible to provide a (meth)acrylamide compound with which a cured product in which an increase in water absorption rate is suppressed can be obtained, a monomer composition containing the (meth)acrylamide compound, a composition for a dental material, and a dental material.

According to Embodiment A-1 of the disclosure, it is possible to provide a (meth)acrylamide compound having a novel structure, a monomer composition containing the (meth)acrylamide compound, a composition for a dental material, a cured product, and a dental material.

### DESCRIPTION OF EMBODIMENTS

In the disclosure, a numerical range that has been indicated by use of "to" indicates the range that includes the numerical values which are described before and after "to", as a lower limit value and an upper limit value, respectively.

In the disclosure, the term "step" includes not only an independent step but also a step by which an intended action of the step is achieved, though the step cannot be clearly distinguished from other steps.

In a case in which a plurality of substances corresponding to each component are present in a composition, the content of each component in the composition in the disclosure means the total content of the plurality of substances present in the composition unless otherwise specified.

In a numerical range described in a stepwise manner in the disclosure, an upper limit value or a lower limit value described in one numerical range may be replaced with an upper limit value or a lower limit value described in another numerical range described in a stepwise manner. In a numerical range described in the disclosure, an upper limit value or a lower limit value of the numerical range may be replaced with a value shown in Examples.

In the disclosure, "light" is a concept including active energy rays such as ultraviolet rays or visible rays.

In the disclosure, "(meth)acrylic compound" means an acrylic compound or a methacrylic compound, "(meth)acryloyl group" means an acryloyl group or a methacryloyl group, "(meth)acrylate" means acrylate or methacrylate, and "(meth)acrylic acid" means acrylic acid or methacrylic acid.

In the disclosure, "iso(thio)cyanate" means isocyanate or isothiocyanate.

The disclosure includes, for example, Embodiment A, and also includes Embodiment A-1 which is a specific embodiment of Embodiment A.

Hereinafter, Embodiment A and Embodiment A-1 will be described in detail.

### [Embodiment A]

### <(Meth)Acrylamide Compound (A)>

A (meth)acrylamide compound (A) includes a cyclic structure and a (meth)acrylamide group.

The (meth)acrylamide compound (A) can be produced, for example, by reacting a (meth)acrylic compound (X), which is at least one selected from the group consisting of a (meth)acrylic acid and a halide of a (meth)acrylic acid with a primary amine compound (Y) including two amino groups and a cyclic structure.

The (meth)acrylamide compound (A) is preferably a reaction product of a (meth)acrylic compound (X), which is at least one selected from the group consisting of a (meth)acrylic acid and a halide of a (meth)acrylic acid and a primary amine compound (Y) including two amino groups and a cyclic structure.

In the primary amine compound (Y), the cyclic structure is not particularly limited.

For example, the cyclic structure may be an alicyclic structure or an aromatic structure, but is preferably an alicyclic structure.

The number of carbon atoms in the cyclic structure is preferably from 4 to 15 and more preferably from 6 to 13. As for the "number of carbon atoms in the cyclic structure", a case in which a hydrogen atom bonded to a carbon atom forming a ring is substituted with a carbon atom, the number of substituted carbon atoms is also included in the "number of carbon atoms in the cyclic structure".

Examples of the case in which a hydrogen atom bonded to a carbon atom forming a ring is substituted with a carbon atom include a case in which a methyl group is bonded to a carbon atom forming a ring.

The primary amine compound (Y) may include one cyclic structure or two or more cyclic structures.

In the primary amine compound (Y), the cyclic structure and two amino groups may be bonded directly or via a divalent linking group.

Examples of the divalent linking group include a methylene group and an ethylene group, and a methylene group is preferable.

The primary amine compound (Y) preferably includes a compound represented by any one of the following Formulas (1-1) to (1-7).

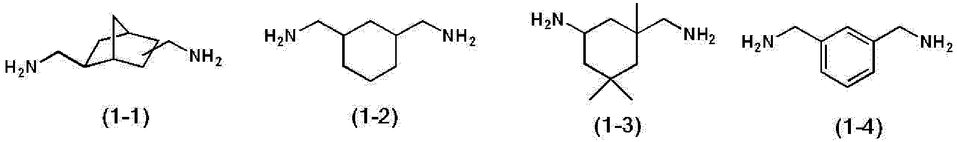

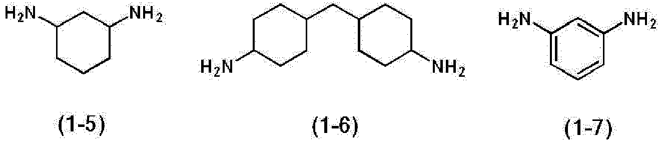

The (meth)acrylamide compound (A) is preferably a compound represented by the following Formula (1).

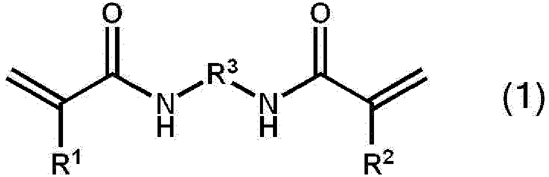

In Formula (1), each of R¹ and R² independently represents a hydrogen atom or a methyl group, and R³ represents a divalent group including a cyclic structure.

In the monomer composition for a dental material of Embodiment A, R³ in Formula (1) is preferably a group represented by the following Formula (1a).

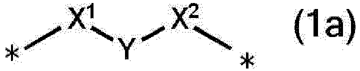

In Formula (1a), each of X¹ and X² independently represents a single bond or a methylene group, Y represents a divalent linking group having from 6 to 13 carbon atoms including an alicyclic structure or an aromatic structure, and each of two *'s represents a bonding position.

In Formula (1a), the divalent linking group in Y preferably has from 6 to 10 carbon atoms and more preferably from 6 to 8 carbon atoms.

The molecular weight of the (meth)acrylamide compound (A) is preferably from 150 to 500, more preferably from 200 to 400, and still more preferably from 200 to 350.

### <<Monomer Composition for Dental Material>>

The monomer composition for a dental material of Embodiment A contains a (meth)acrylamide compound (A) including a cyclic structure and a (meth)acrylamide group.

When the monomer composition for a dental material of Embodiment A contains the (meth)acrylamide compound (A), a cured product can be obtained in which an increase in water absorption rate is suppressed. As a result, since a cured product can be obtained in which mechanical properties are maintained even in the oral cavity, the monomer composition for a dental material is suitable for a dental material.

Since the cured product in Embodiment A has particularly excellent bending strength, the elastic modulus and breaking strength of the cured product can be favorably maintained, for example.

In general, in a case in which a cured product obtained using a monomer composition for a dental material contains a monomer for improving adhesive strength, the water absorption rate tends to be high, and mechanical properties may be deteriorated owing to an increase in water absorption rate.

On the other hand, in the monomer composition for a dental material of Embodiment A, by using the (meth)acrylamide compound (A) including a cyclic structure and a (meth)acrylamide group as a monomer, it is also possible to suppress an increase in the water absorption rate while increasing the adhesive strength of the resulting cured product relative to a tooth substance.

That is, the monomer composition for a dental material of Embodiment A can obtain a cured product in which an increase in the water absorption rate is suppressed and which has excellent adhesive strength relative to a tooth substance.

The monomer composition for a dental material of Embodiment A may contain a component other than the (meth)acrylamide compound (A), but it is preferable that the monomer composition for a dental material does not substantially contain a component other than the (meth)acrylamide compound (A).

Thus, the timing of commencement of the polymerization can be adjusted. That is, in a case in which the monomer composition for a dental material does not substantially contain a component other than the (meth)acrylamide compound (A), curing is not started, and polymerization can be started by adding a polymerization initiator at a desired time.

From the above viewpoint, for example, the content of the (meth)acrylamide compound (A) is preferably 90% by mass or more and more preferably 95% by mass or more with respect to the total mass of the monomer composition for a dental material. The content of the (meth)acrylamide compound (A) may be 99% by mass or less with respect to the total mass of the monomer composition for a dental material.

### <Composition for Dental Material>

The composition for a dental material of Embodiment A contains the monomer composition for a dental material of Embodiment A and a polymerization initiator.

The content of the monomer composition for a dental material is not particularly limited, but is preferably 5% by mass or more and more preferably 10% by mass or more with respect to the total mass of the composition for a dental material. The content of the monomer composition for a dental material is preferably 30% by mass or less with respect to the total mass of the composition for a dental material.

### <Polymerization Initiator>

The composition for a dental material of Embodiment A contains a polymerization initiator.

In a case in which the composition for a dental material of Embodiment A contains a polymerization initiator, the polymerization initiator to be contained may be only one kind or two or more kinds.

In a case in which the composition for a dental material of Embodiment A contains a polymerization initiator, the polymerization of a monomer (that is, the (meth)acrylamide compound (A) and another monomer contained if necessary; the same applies hereinafter) can be further promoted in the process of curing the composition for a dental material.

As a polymerization initiator in a case in which room temperature polymerization is performed for polymerization of monomers, for example, a redox-based polymerization initiator obtained by combining an oxidizing agent and a reducing agent is preferable.

In a case in which a redox-based polymerization initiator is used, for example, an oxidizing agent and a reducing agent in separately packaged forms may be prepared, and the two may be mixed immediately before use.

The oxidizing agent is not particularly restricted, and examples thereof include organic peroxides such as a diacyl peroxide (such as benzoyl peroxide), a peroxy ester (such as t-butyl peroxy benzoate), a dialkyl peroxide (such as dicumyl peroxide), a peroxyketal (such as 1,1-bis(t-butylperoxy)-3,3,5-trimethylcyclohexane), a ketone peroxide (such as methyl ethyl ketone peroxide), and a hydroperoxide (t-butyl hydroperoxide).

The reducing agent is not particularly limited, and a tertiary amine (such as N,N-dimethylaniline) is usually used.

In addition to these organic peroxide/amine-based polymerization initiators, a redox-based polymerization initiator such as a cumene hydroperoxide/thiourea-based polymerization initiator, an ascorbic acid/Cu²⁺ salt-based polymerization initiator, or an organic peroxide/amine/sulfinic acid (or a salt thereof)-based polymerization initiator can be used.

As a polymerization initiator, tributylborane, organic sulfinic acid, or the like is also suitably used.

In a case in which thermal polymerization by heating is performed for polymerization of monomers, a peroxide, an azo-based compound, or the like is preferable as a polymerization initiator.

The peroxide is not particularly limited, and examples thereof include benzoyl peroxide, t-butyl hydroperoxide, and cumene hydroperoxide.

The azo-based compound is not particularly limited, and examples thereof include azobisisobutyronitrile.

As a polymerization initiator (hereinafter, also referred to as "photopolymerization initiator") in a case in which photopolymerization by visible light irradiation is performed for polymerization of monomers, a redox-based initiator such as α-diketone/tertiary amine, α-diketone/aldehyde, or α-diketone/mercaptan is preferable.

The photopolymerization initiator is not particularly limited, and examples thereof include an α-diketone/reducing agent, a ketal/reducing agent, and a thioxanthone/reducing agent.

Examples of the α-diketone include camphorquinone.

Examples of the ketal include benzyl dimethyl ketal.

Examples of the thioxanthone include 2-chlorothioxanthone.

Examples of the reducing agent include a tertiary amine (such as Michler Ketone), an aldehyde (such as citronellal), and a compound having a thiol group (such as 2-mercaptobenzoxazole).

An initiator such as an α-diketone/organic peroxide/reducing agent or the like-based initiator, in which an organic peroxide is added to these redox-based initiators, is also suitably used.

In a case in which photopolymerization is performed by ultraviolet irradiation, a photopolymerization initiator such as benzoin alkyl ether or benzyl dimethyl ketal is preferable. Photopolymerization initiators such as (bis)acylphosphine oxides are also suitably used.

Examples of the (bis)acylphosphine oxides include acylphosphine oxides (such as 2,4,6-trimethylbenzoyl diphenylphosphine oxide), and bisacylphosphine oxides (such as bis-(2,6-dichlorobenzoyl)phenylphosphine oxide).

These (bis)acylphosphine oxide photopolymerization initiators may also be used singly or in combination with a reducing agent such as various amines, aldehydes, mercaptans, or sulfinates.

These (bis)acylphosphine oxide photopolymerization initiators may be used in combination with the photopolymerization initiator of visible light.

In addition to these polymerization initiators, co-catalyst components such as an amine compound such as dimethylaminoethyl methacrylate, N,N-dimethyl-p-toluidine, ethyl p-dimethylaminobenzoate (also referred to as EDB), or 2-butoxyethyl 4-(dimethylamino) benzoate (also referred to as DMBE), an aldehyde compound such as citronellal or dimethylaminobenzaldehyde, and a compound having a thiol group such as 2-mercaptobenzoxazole or decanethiol may be used in combination.

For example, a polymerization initiator may be used with reference to WO 2019/107323 A1, WO 2020/040141 A1, or the like.

The content of the polymerization initiator is preferably from 0.01% by mass to 20% by mass and more preferably from 0.1% by mass to 5% by mass with respect to the total amount of monomers contained in the composition for a dental material.

### <Other Monomers>

The composition for a dental material of Embodiment A may contain a monomer other than the monomer (mainly, the (meth)acrylamide compound (A)) contained in the monomer composition for dental material.

Examples of the other monomer include the following (meth)acrylate compound (B).

### <(Meth)Acrylate Compound (B)>

The composition for a dental material of Embodiment A preferably further contains a (meth)acrylate compound (B) other than the (meth)acrylamide compound (A).

The (meth)acrylate compound (B) can be used without particular limitation as long as it is a (meth)acrylate compound other than the (meth)acrylamide compound (A).

Specific examples of the (meth)acrylate compound (B) include a (meth)acrylate compound containing two or more (meth)acryloyloxy groups and a (meth)acrylate compound containing one (meth)acryloyloxy group.

Hereinafter, specific examples of the (meth)acrylate compound (B) will be described.

### ((Meth)Acrylate Compound Containing Two or More (Meth)Acryloyloxy Groups)

The (meth)acrylate compound (B) may include a (meth)acrylate compound containing two or more (meth)acryloyloxy groups.

Examples of the (meth)acrylate compound containing two or more (meth)acryloyloxy groups include a bifunctional (meth)acrylate compound and a tri- or higher functional (meth)acrylate compound.

Examples of the bifunctional (meth)acrylate compound include an aromatic compound-based bifunctional (meth)acrylate compound, an aliphatic compound-based bifunctional (meth)acrylate compound, a (meth)acrylate compound including two or more urethane bonds, and a (meth)acrylate compound including two or more thiourethane bonds.

Examples of the aromatic compound-based bifunctional (meth)acrylate compound include 2,2-bis((meth)acryloyloxyphenyl)propane, 2,2-bis[4-(3-(meth)acryloyloxy)-2-hydroxypropoxyphenyl]propane, 2,2-bis(4-(meth)acryloyloxyethoxyphenyl)propane, 2,2-bis(4-(meth)acryloyloxypolyethoxyphenyl)propane, and 2,2-bis(4-(meth)acryloyloxypolypropylene phenyl)propane.

Among them, at least one selected from the group consisting of 2,2-bis[4-(3-methacryloyloxy)-2-hydroxypropoxyphenyl]propane (also referred to as Bis-GMA) and 2,2-bis(4-methacryloyloxypolyethoxyphenyl)propane is preferable.

Examples of the aliphatic compound-based bifunctional (meth)acrylate compound include an aliphatic compound-based bifunctional (meth)acrylate compound not including a urethane bond and a thiourethane bond, a (meth)acrylate compound including two or more urethane bonds, and a (meth)acrylate compound including two or more thiourethane bonds.

Examples of the aliphatic compound-based bifunctional (meth)acrylate compound not including a urethane bond and a thiourethane bond include erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol di(meth)acrylate (also referred to as 3G), nonaethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, and 1,2-bis(3-methacryloyloxy-2-hydroxypropyloxy)ethane.

Among them, at least one selected from the group consisting of glycerol dimethacrylate, triethylene glycol dimethacrylate, 1,6-hexanediol dimethacrylate (also referred to as HexDMA), neopentyl glycol dimethacrylate (also referred to as NPG), and 1,2-bis(3-methacryloyloxy-2-hydroxypropyloxy)ethane is preferable.

The (meth)acrylate compound including two or more urethane bonds can be produced, for example, as a reaction product of an iso(thio)cyanate compound containing two or more iso(thio)cyanate groups and a (meth)acrylate compound containing a hydroxy group described below.

In the (meth)acrylate compound including two or more urethane bonds, the number of urethane bonds is preferably 2.

The (meth)acrylate compound including two or more urethane bonds is not particularly limited. Examples thereof include 2,2,4-trimethylhexamethylene bis(2-carbamoyloxyethyl) dimethacrylate (also referred to as UDMA).

The (meth)acrylate compound including two or more thiourethane bonds can be produced, for example, as a reaction product of a thiol compound containing two or more thiol groups (such as pentaerythritol tetrakis(3-mercaptopropionate)), an iso(thio)cyanate compound containing two or more iso(thio)cyanate groups, and a (meth)acrylate compound containing a hydroxy group.

In Embodiment A, the "(meth)acrylate compound including two or more urethane bonds" does not have two or more thiourethane bonds.

In Embodiment A, the "(meth)acrylate compound including two or more thiourethane bonds" may have two or more urethane bonds.

Examples of the tri- or higher functional (meth)acrylate compound include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene) bis[2-(aminocarboxy)propane-1,3-diol] tetramethacrylate, and 1,7-diacryloyloxy-2,2,6,6-tetraacryloyloxymethyl-4-oxyheptane.

### (Iso(Thio)Cyanate Compound)

Examples of the iso(thio)cyanate compound containing two or more iso(thio)cyanate groups that can be used as a raw material of the (meth)acrylate compound including two or more urethane bonds or thiourethane bonds include the following isocyanate compound and isothiocyanate compound.

Examples of the isocyanate compound include hexamethylene diisocyanate, 2,2,4-trimethylhexamethylene diisocyanate, 2,4,4-trimethylhexamethylene diisocyanate, pentamethylene diisocyanate, m-xylylene diisocyanate, 1,3-tetramethylxylylene diisocyanate, isophorone diisocyanate, bis(isocyanatemethyl)cyclohexane, bis(isocyanatecyclohexyl)methane, 2,5-bis(isocyanatemethyl)bicyclo-[2.2.1]-heptane, 2,6-bis(isocyanatemethyl)bicyclo-[2.2.1]-heptane, tolylene diisocyanate, phenylene diisocyanate, and 4,4'-diphenylmethane diisocyanate.

As the isocyanate compound, one kind may be used, or two or more kinds may be combined.

Examples of the isothiocyanate compound include an alicyclic polyisothiocyanate compound such as hexamethylene diisothiocyanate; an aromatic polyisothiocyanate compound such as tolylene diisothiocyanate; and a sulfur-containing heterocyclic polyisothiocyanate compound such as 2,5-diisothiocyanate thiophene.

As the isothiocyanate compound, one kind may be used, or two or more kinds may be combined.

Among the above, the iso(thio)cyanate compound preferably includes at least one selected from the group consisting of hexamethylene diisocyanate, 2,2,4-trimethylhexamethylene diisocyanate, 2,4,4-trimethylhexamethylene diisocyanate, pentamethylene diisocyanate, m-xylylene diisocyanate, 1,3-tetramethylxylylene diisocyanate, isophorone diisocyanate, bis(isocyanatemethyl)cyclohexane, bis(isocyanatecyclohexyl)methane, 2,5-bis(isocyanatemethyl)bicyclo-[2.2.1]-heptane, 2,6-bis(isocyanatemethyl)bicyclo-[2.2.1]-heptane, tolylene diisocyanate, phenylene diisocyanate, and 4,4'-diphenylmethane diisocyanate.

### ((Meth)Acrylate Compound Containing Hydroxy Group)

Examples of the (meth)acrylate compound containing a hydroxy group that can be used as a raw material of the (meth)acrylate compound including two or more urethane bonds or thiourethane bonds include 2-hydroxyethyl (meth)acrylate (also referred to as HEMA), 2-hydroxypropyl (meth)acrylate, 2-hydroxybutyl (meth)acrylate, and 4-hydroxybutyl (meth)acrylate.

Among the above, the (meth)acrylate compound containing a hydroxy group preferably includes at least one selected from the group consisting of 2-hydroxyethyl (meth)acrylate and 2-hydroxypropyl (meth)acrylate.

### ((Meth)Acrylate Compound Containing One (Meth)Acryloyloxy Group)

Examples of the (meth)acrylate compound containing one (meth)acryloyloxy group in the (meth)acrylate compound (B) of Embodiment A include a (meth)acrylate compound containing a hydroxy group and a (meth)acrylate compound not including a hydroxy group.

Examples of the (meth)acrylate compound containing a hydroxy group include 2-hydroxyethyl (meth)acrylate, 2-hydroxypropyl (meth)acrylate, 2-hydroxybutyl (meth)acrylate, 4-hydroxybutyl (meth)acrylate.

Among the above, the (meth)acrylate compound containing a hydroxy group preferably includes at least one selected from the group consisting of 2-hydroxyethyl (meth)acrylate and 2-hydroxypropyl (meth)acrylate.

Examples of the (meth)acrylate compound not including a hydroxy group include alkyl (meth)acrylate such as dodecyl (meth)acrylate (DMA).

### ((Meth)Acrylate Compound Containing Acidic Group)

The (meth)acrylate compound (B) may include a (meth)acrylate compound containing an acidic group.

The (meth)acrylate compound containing an acidic group has affinity with an adherend and also has a decalcification effect on a tooth substance.

Examples of the acidic group include a phosphate group, a pyrophosphate group, a thiophosphate group, a phosphonate group, a sulfonic acid group, and a carboxylic acid group.

Among the above, the acidic group is preferably at least one selected from the group consisting of a phosphate group and a carboxylic acid group, and more preferably a phosphate group.

Examples of the (meth)acrylate compound containing a phosphate group include (meth)acryloyloxyalkyl dihydrogen phosphate such as 10-(phosphonooxy)decyl-(meth)acrylate (also referred to as MDP), bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethyl phenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, and acid chlorides thereof, alkali metal salts, and ammonium salts thereof.

Examples of the (meth)acrylate compound containing a carboxylic acid group include 4-methacryloyloxyethyltrimellitic acid (also referred to as 4-MET) and an anhydride thereof.

### <Filler>

The composition for a dental material of Embodiment A may contain a filler. The filler can be used without particular limitation as long as it is a general filler used in the field of dentistry. The filler is broadly classified into an organic filler and an inorganic filler.

Examples of the organic filler include fine powders of polymethyl methacrylate, a methyl methacrylate-ethyl methacrylate copolymer, an ethylene-vinyl acetate copolymer, a styrene-butadiene copolymer, and the like.

Examples of the inorganic filler include fine powders of various types of glasses (mainly composed of silicon dioxide and, if necessary, containing oxides of heavy metals, boron, aluminum, or the like), various ceramics, diatomaceous earth, kaolin, clay minerals (such as montmorillonite), activated clay, synthetic zeolite, mica, calcium fluoride, ytterbium fluoride, calcium phosphate, barium sulfate, zirconium dioxide, titanium dioxide, hydroxyapatite, and the like. Specific examples of such an inorganic filler include barium borosilicate glass (such as SCHOTT 8235, SCHOTT GM27884, or SCHOTT G018-053), strontium boroaluminosilicate glass (such as SCHOTT G018-163), lanthanum glass (such as SCHOTT GM31684), fluoroaluminosilicate glass (such as SCHOTT G018-117), and boroaluminosilicate glass containing zirconium, cesium, or the like (such as SCHOTT G018-307).

These fillers may be used singly or in combination of two or more kinds thereof. The content of the filler may be appropriately determined in consideration of the operability (consistency) of the composition for a dental material (for example, a composite resin composition), the bending strength of the cured product, and the like, and is preferably from 10 parts by mass to 2000 parts by mass, more preferably from 50 parts by mass to 1000 parts by mass, and still more preferably from 100 parts by mass to 600 parts by mass with respect to 100 parts by mass of all components other than the filler contained in the composition for a dental material.

### <Solvent>

The composition for a dental material of Embodiment A preferably contains a solvent.

Examples of the solvent include an organic solvent and water.

The composition for a dental material of Embodiment A preferably contains an organic solvent and water.

As the organic solvent, ethanol, isopropyl alcohol, acetone, or the like can be used.

Examples of water include distilled water and ultrapure water.

### <Dental Material>

The dental material of Embodiment A contains a cured product of the composition for a dental material of Embodiment A.

The composition for a dental material of Embodiment A can be used for a dental material application.

Examples of the dental material include a dental restorative material, a denture base resin, a denture base lining material, an impression material, a joint wearing material (such as resin cement or resin-added glass ionomer cement), a dental adhesive (such as an orthodontic adhesive or a cavity coating adhesive), a tooth fissure sealant, a resin block for CAD/CAM, temporary crown, and an artificial tooth material.

Examples of the dental restorative material include a composite resin for a crown, a composite resin for filling a carious cavity, a composite resin for abutment construction, and a composite resin for filling restoration.

### Embodiment A of the disclosure includes the following aspects.

<1> A monomer composition for a dental material containing a (meth)acrylamide compound (A) including a cyclic structure and a (meth)acrylamide group.
<2> The monomer composition for a dental material according to <1>, in which the (meth)acrylamide compound (A) is a reaction product of a (meth)acrylic compound (X), which is at least one selected from the group consisting of a (meth)acrylic acid and a halide of a (meth)acrylic acid, and a primary amine compound (Y) including two amino groups and a cyclic structure.
<3> The monomer composition for a dental material according to<2>, in which the primary amine compound (Y) includes a compound represented by any one of the following Formulas (1-1) to (1-7): 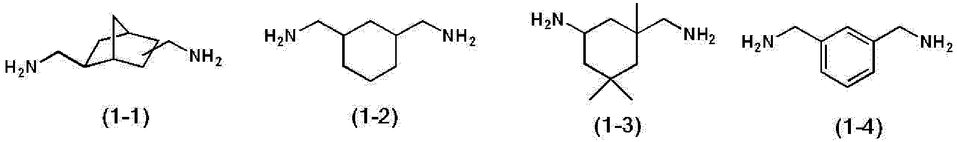 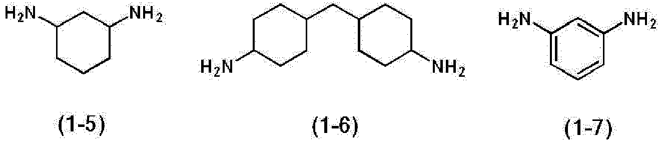
<4> The monomer composition for a dental material according to any one of <1> to <3>, in which the (meth)acrylamide compound (A) is a compound represented by the following Formula (1). 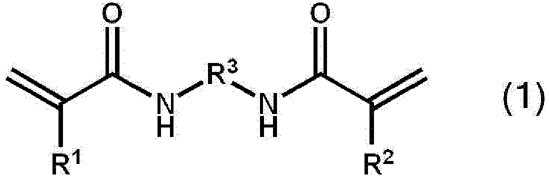 In Formula (1), each of R¹ and R² independently represents a hydrogen atom or a methyl group, and R³ is a divalent group including a cyclic structure.
<5> The monomer composition for a dental material according to <4>, in which the R³ in Formula (1) is a group represented by the following Formula (1a). 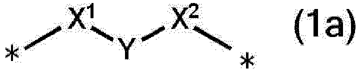 In Formula (1a), each of X¹ and X² independently represents a single bond or a methylene group, Y represents a divalent linking group having from 6 to 13 carbon atoms including an alicyclic structure or an aromatic structure, and each of two *'s represents a bonding position.
<6> The monomer composition for a dental material according to any one of <1> to <5>, in which a content of the (meth)acrylamide compound (A) is 90% by mass or more with respect to the total mass of the monomer composition for a dental material.
<7> A composition for a dental material containing the monomer composition for a dental material according to any one of <1> to <6> and a polymerization initiator.
<8> A dental material containing a cured product of the composition for a dental material according to <7>.

### [Embodiment A-1]

### «(Meth)Acrylamide Compound (A)»

The (meth)acrylamide compound (A) of Embodiment A-1 (also referred to as the (meth)acrylamide compound (A-1) in the disclosure) contains two (meth)acrylamide groups and a divalent alicyclic hydrocarbon group, in which a nitrogen atom in each of the two (meth)acrylamide groups isbonded to the divalent alicyclic hydrocarbon group via a methylene group.

The (meth)acrylamide compound (A-1) of Embodiment A-1 has a novel structure.

A cured product obtained by polymerizing the (meth)acrylamide compound (A-1) of Embodiment A-1 is excellent in adhesive strength to a tooth substance such as an enamel.

In the cured product obtained by polymerizing the (meth)acrylamide compound (A-1) of Embodiment A-1, an increase in water absorption rate is suppressed. As a result, for example, since mechanical properties are maintained even in the oral cavity, the (meth)acrylamide compound (A-1) is suitable for a dental material.

Since the cured product obtained by polymerizing the (meth)acrylamide compound (A-1) of Embodiment A-1 is particularly excellent in bending strength, for example, the elastic modulus and breaking strength of the cured product can be favorably maintained.

In general, in a case in which a cured product obtained using the monomer composition for a dental material contains a monomer for improving an adhesive strength, the water absorption rate tends to be high, and mechanical properties may be deteriorated due to an increase in water absorption rate.

On the other hand, the monomer composition for a dental material of Embodiment A-1 is not only excellent in adhesive strength of the resulting cured product to a tooth substance, but also can suppress an increase in water absorption rate.

The (meth)acrylamide compound (A-1) of Embodiment A-1 can be produced, for example, by reacting a (meth)acrylic compound (X), which is at least one selected from the group consisting of a (meth)acrylic acid and a halide of a (meth)acrylic acid, with a primary amine compound (Y1) that includes two amino groups and the divalent alicyclic hydrocarbon group, in which a nitrogen atom in each of the two amino groups is bonded to the divalent alicyclic hydrocarbon group via a methylene group.

The (meth)acrylamide compound of Embodiment A-1 is preferably a reaction product of: a (meth)acrylic compound (X) which is at least one selected from the group consisting of a (meth)acrylic acid and a halide of a (meth)acrylic acid, and a primary amine compound (Y1) that includes two amino groups and the divalent alicyclic hydrocarbon group, in which a nitrogen atom in each of the two amino groups is bonded to the divalent alicyclic hydrocarbon group via a methylene group.

In the primary amine compound (Y1), the alicyclic hydrocarbon group is not particularly limited.

The number of carbon atoms of the alicyclic hydrocarbon group is preferably from 4 to 15, more preferably from 6 to 13, still more preferably from 6 to 10, and particularly preferably from 6 to 8. As for the "number of carbon atoms of the alicyclic hydrocarbon group", a case in which a hydrogen atom bonded to a carbon atom forming a ring is substituted with a carbon atom, the number of substituted carbon atoms is also included in the "number of carbon atoms of the alicyclic hydrocarbon group".

Examples of the case in which a hydrogen atom bonded to a carbon atom forming a ring is substituted with a carbon atom include a case in which a methyl group is bonded to a carbon atom forming a ring.

The primary amine compound (Y1) may include one alicyclic hydrocarbon group or two or more alicyclic hydrocarbon groups.

The primary amine compound (Y1) preferably includes a compound represented by the following Formula (1-1) or (1-2):

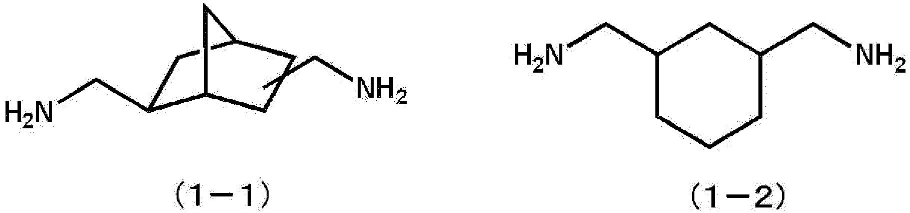

The (meth)acrylamide compound (A-1) is preferably a compound represented by the following Formula (1A).

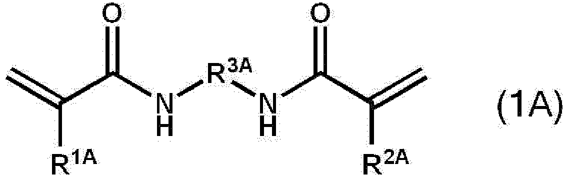

In Formula (1A), each of R^{1A} and R^{2A} independently represents a hydrogen atom or a methyl group, and R^{3A} represents a divalent group formed by linking a methylene group, a divalent alicyclic hydrocarbon group, and a methylene group in this order.

In the (meth)acrylamide compound (A-1) of Embodiment A-1, R^{3A} in Formula (1A) is preferably a group represented by the following Formula (1Aa).

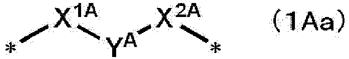

In Formula (1Aa), each of X^{1A} and X^{2A} independently represents a methylene group, Y^{A} is a divalent alicyclic hydrocarbon group having from 6 to 9 carbon atoms, and each of two *'s represents a bonding position.

In Formula (1Aa), the divalent linking group in Y^{A} preferably has from 6 to 10 carbon atoms and more preferably from 6 to 8 carbon atoms.

The molecular weight of the (meth)acrylamide compound (A-1) is preferably from 150 to 500, more preferably from 200 to 400, and still more preferably from 200 to 350.

### <<Monomer Composition>>

The monomer composition of Embodiment A-1 contains the (meth)acrylamide compound (A-1) of Embodiment A-1.

The monomer composition of Embodiment A-1 may contain a component other than the (meth)acrylamide compound (A-1), but it is preferable that the monomer composition does not substantially contain a component other than the (meth)acrylamide compound (A-1).

Thus, the timing of starting the polymerization can be adjusted. That is, in a case in which the monomer composition does not substantially contain a component other than the (meth)acrylamide compound (A-1), curing is not started, and polymerization can be started by adding a polymerization initiator at a desired time.

From the above viewpoint, for example, the content of the (meth)acrylamide compound (A-1) is preferably 90% by mass or more and more preferably 95% by mass or more with respect to the total mass of the monomer composition. The content of the (meth)acrylamide compound (A-1) may be 99% by mass or less with respect to the total mass of the monomer composition.

The monomer composition of Embodiment A-1 is preferably used for a dental material.

When the monomer composition of Embodiment A-1 contains the (meth)acrylamide compound (A-1) of Embodiment A-1, a cured product in which an increase in water absorption rate is suppressed can be obtained. As a result, a cured product in which mechanical properties are maintained can be obtained.

In the cured product in Embodiment A-1, particularly, since the bending strength is favorably maintained, for example, the elastic modulus and breaking strength of the cured product can be favorably maintained.

In general, in a case in which a cured product obtained using the monomer composition has increased adhesive strength to a tooth substance such as an enamel or dentin, mechanical properties may be deteriorated due to an increase in water absorption rate.

By using the (meth)acrylamide compound (A-1) of Embodiment A-1 as a monomer, it is also possible to increase the adhesive strength of the resulting cured product to a tooth substance.

That is, the monomer composition of Embodiment A-1 can obtain a cured product which is suppressed in an increase in water absorption rate and is excellent in adhesive strength to a tooth substance.

### <Composition for Dental Material>

The composition for a dental material of Embodiment A-1 contains the monomer composition of Embodiment A-1 and a polymerization initiator.

The content of the monomer composition is not particularly limited, but is preferably 5% by mass or more and more preferably 10% by mass or more with respect to the total mass of the composition for a dental material. The content of the monomer composition is preferably 30% by mass or less with respect to the total mass of the composition for a dental material.

### <Polymerization Initiator>

The composition for a dental material of Embodiment A-1 contains a polymerization initiator.

In a case in which the composition for a dental material of Embodiment A-1 contains a polymerization initiator, the polymerization initiator to be contained may be only one kind or two or more kinds.

In a case in which the composition for a dental material of Embodiment A-1 contains a polymerization initiator, the polymerization of a monomer (that is, the (meth)acrylamide compound (A-1) and another monomer contained if necessary; the same applies hereinafter) can be further promoted in the process of curing the composition for a dental material.

As a polymerization initiator in a case in which room temperature polymerization is performed for polymerization of monomers, for example, a redox-based polymerization initiator obtained by combining an oxidizing agent and a reducing agent is preferable.

In a case in which a redox-based polymerization initiator is used, for example, an oxidizing agent and a reducing agent in separately packaged forms may be prepared, and the two may be mixed immediately before use.

Details of specific examples, preferred specific examples, preferred content, preferred aspects, co-catalyst components, and the like of the polymerization initiator are the same as details of specific examples, preferred specific examples, preferred content, preferred aspects, co-catalyst components, and the like of the polymerization initiator in Embodiment A.

### <Other Monomers>

The composition for a dental material of Embodiment A-1 may contain a monomer other than the monomer (mainly, the (meth)acrylamide compound (A-1)) contained in the dental material monomer composition.

Examples of the other monomer include the following (meth)acrylate compound (B).

### <(Meth)Acrylate Compound (B)>

The composition for a dental material of Embodiment A-1 preferably further contains a (meth)acrylate compound (B) other than the (meth)acrylamide compound (A-1).

The (meth)acrylate compound (B) can be used without particular limitation as long as it is a (meth)acrylate compound other than the (meth)acrylamide compound (A-1).

Specific examples of the (meth)acrylate compound (B) include a (meth)acrylate compound containing two or more (meth)acryloyloxy groups and a (meth)acrylate compound containing one (meth)acryloyloxy group.

Hereinafter, specific examples of the (meth)acrylate compound (B) will be described.

Details of specific examples, preferred specific examples, preferred aspects, and the like of the (meth)acrylate compound (B) are the same as the details of specific examples, preferred specific examples, preferred aspects, and the like of the (meth)acrylate compound (B) in Embodiment A.

### ((Meth)Acrylate Compound Containing Two or More (Meth)Acryloyloxy Groups)

Details of specific examples, preferred specific examples, preferred aspects, and the like of the (meth)acrylate compound containing two or more (meth)acryloyloxy groups are the same as the details of specific examples, preferred specific examples, preferred aspects, and the like of the (meth)acrylate compound containing two or more (meth)acryloyloxy groups in Embodiment A.

### ((Meth)Acrylate Compound Containing One (Meth)Acryloyloxy Group)

Details of specific examples, preferred specific examples, preferred aspects, and the like of the (meth)acrylate compound containing one (meth)acryloyloxy group are the same as the details of specific examples, preferred specific examples, preferred aspects, and the like of the (meth)acrylate compound containing one (meth)acryloyloxy group in Embodiment A.

### ((Meth)Acrylate Compound Containing Acidic Group)

The (meth)acrylate compound (B) may include a (meth)acrylate compound containing an acidic group.

Details of specific examples, preferred specific examples, preferred aspects, and the like of the (meth)acrylate compound containing an acidic group are the same as the details of specific examples, preferred specific examples, preferred aspects, and the like of the (meth)acrylate compound containing an acidic group in Embodiment A.

### <Filler>

The composition for a dental material of Embodiment A-1 may contain a filler. The filler can be used without particular limitation as long as it is a general filler used in the field of dentistry. The filler is broadly classified into an organic filler and an inorganic filler.

Details of specific examples, preferred specific examples, preferred aspects, and the like of the filler are the same as the details of specific examples, preferred specific examples, preferred aspects, and the like of the filler in Embodiment A.

### <Solvent>

The composition for a dental material of Embodiment A-1 preferably contains a solvent.

Examples of the solvent include an organic solvent and water.

The composition for a dental material of Embodiment A-1 preferably contains an organic solvent and water.

As the organic solvent, ethanol, isopropyl alcohol, acetone, or the like can be used.

Examples of water include distilled water and ultrapure water.

### <<Cured Product>>

The cured product of Embodiment A-1 is a cured product of the composition for a dental material of Embodiment A-1.

The cured product of Embodiment A-1 is excellent in adhesive strength to a tooth substance such as an enamel.

In the cured product of Embodiment A-1, an increase in water absorption rate is suppressed. As a result, the bending strength is maintained.

In the cured product of Embodiment A-1, since the bending strength is favorably maintained, for example, the elastic modulus and breaking strength of the cured product can be favorably maintained.

### <Dental Material>

The dental material of Embodiment A-1 contains a cured product of the composition for a dental material of Embodiment A-1.

The composition for a dental material of Embodiment A-1 can be used for a dental material application.

Examples of the dental material include a dental restorative material, a denture base resin, a denture base lining material, an impression material, a joint wearing material (such as resin cement or resin-added glass ionomer cement), a dental adhesive (such as an orthodontic adhesive or a cavity coating adhesive), a tooth fissure sealant, a resin block for CAD/CAM, temporary crown, and an artificial tooth material.

Examples of the dental restorative material include a composite resin for a crown, a composite resin for filling a carious cavity, a composite resin for abutment construction, and a composite resin for filling restoration.

### Means for solving the above problems include the following aspects.

<1> A (meth)acrylamide compound containing two (meth)acrylamide groups and a divalent alicyclic hydrocarbon group, in which nitrogen atoms in the two (meth)acrylamide groups are both bonded to the divalent alicyclic hydrocarbon group via a methylene group.
<2> The (meth)acrylamide compound according to <1>, which is a reaction product of a (meth)acrylic compound (X) which is at least one selected from the group consisting of a (meth)acrylic acid and a halide of a (meth)acrylic acid, and a primary amine compound (Y1) that includes two amino groups and the divalent alicyclic hydrocarbon group, in which nitrogen atoms in the two amino groups are both bonded to the divalent alicyclic hydrocarbon group via a methylene group.
<3> The (meth)acrylamide compound according to <2>, in which the primary amine compound (Y1) includes a compound represented by the following Formula (1-1) or (1-2): [0126] 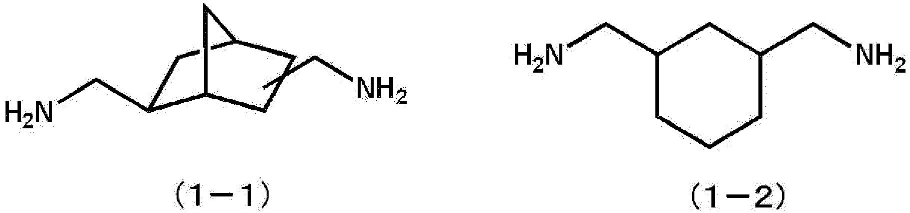
<4> The (meth)acrylamide compound according to any one of <1> to <3>, which is a compound represented by the following Formula (1A). 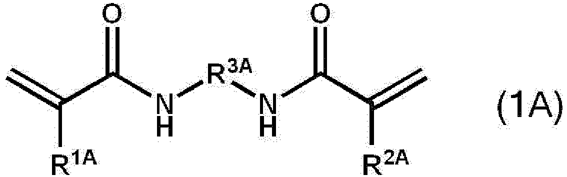 In Formula (1A), each of R^{1A} and R^{2A} independently represents a hydrogen atom or a methyl group, and R^{3A} represents a divalent group formed by linking a methylene group, a divalent alicyclic hydrocarbon group, and a methylene group in this order.
<5> The (meth)acrylamide compound according to <4>, in which the R^{3A} in Formula (1A) is a group represented by the following Formula (1Aa). [0130] 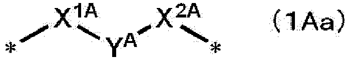 In Formula (1Aa), each of X^{1A} and X^{2A} independently represents a methylene group, Y^{A} represents a divalent alicyclic hydrocarbon group having from 6 to 9 carbon atoms, and two *'s each represent a bonding position.
<6> A monomer composition containing the (meth)acrylamide compound according to any one of <1> to <5>.
<7> The monomer composition according to <6>, in which a content of the (meth)acrylamide compound is 90% by mass or more with respect to the total mass of the monomer composition.
<8> The monomer composition according to <6> or <7>, which is used for a dental material.
<9> A composition for a dental material containing the monomer composition according to any one of <6> to <8> and a polymerization initiator.
<10> A cured product of the composition for a dental material according to <9>.
<11> A dental material containing the cured product according to <10>.

### Examples

Hereinafter, Embodiment A will be more specifically described with reference to Examples; however, Embodiment A is not limited to the following Examples.
Abbreviations of compounds used in Examples of Embodiment A are shown below.
EDC·HCl: 1-(3-dimethylaminopropyl)-3-ethylcarbodiimide hydrochloride
DCM: dichloromethane
NBDA: bis(aminomethyl)norbornane
H6XDA: 1,3-bis(aminomethyl)cyclohexane
IPDA: isophorone diamine
XDA: m-xylylenediamine
CHDA: 1,3-cyclohexanediamine
H12MDA: 4,4'-methylenebis(cyclohexylamine)
mPDA: m-phenylenediamine
D-400: JEFFAMINE (registered trademark) D-400
D-2000: JEFFAMINE (registered trademark) D-2000
DODA: 1,2-bis(2-aminoethoxy)ethane
UDMA: 2,2,4-trimethylhexamethylene bis(2-carbamoyloxyethyl) dimethacrylate
HEMA: 2-hydroxyethyl methacrylate
MDP: 10-(phosphonooxy)decyl-methacrylate
3G: triethylene glycol dimethacrylate
CQ: camphorquinone
DMBE: 2-butoxyethyl 4-(dimethylamino) benzoate
EtOH: ethanol
DW: distilled water

Structural formulas of NBDA, H6XDA, IPDA, XDA, CHDA, H12MDA, mPDA, D-400, D-2000, and DODA are as follows.

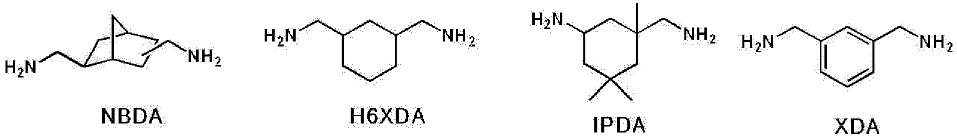

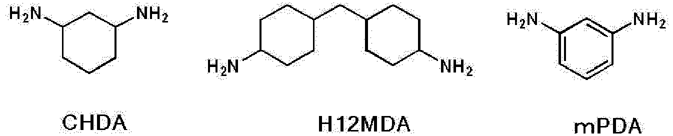

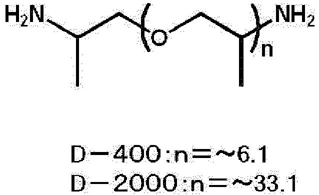

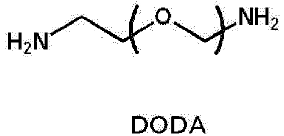

### [Method of Measuring HPLC]

The HPLC chart spectrum of the (meth)acrylamide compound obtained in each Example or Comparative Example was measured using an HPLC apparatus: LC-20AT manufactured by SHIMADZU CORPORATION.

After dissolving the (meth)acrylamide compound obtained in each Example or Comparative Example in CH₃CN, the (meth)acrylamide compound was measured with an eluent of CH₃CN/H₂O = 90/10.

### [Method of Adhesive Strength Test]

A method of an adhesive strength test in Examples and Comparative Examples of Embodiment A is shown below.

### (Preparation of Test Piece for Adhesive Strength Test)

A bovine lower anterior tooth extracted and kept in a frozen state was thawed by injection of water, and subjected to root amputation and pulp extirpation treatment. This was placed in a plastic cylindrical container having a diameter of 25 mm and a depth of 25 mm, and embedded in an acrylic resin. The surface thereof was wet-polished with #120 and #400 emery papers to expose an enamel in a state of being parallel to the lip surface, thereby preparing an enamel adherend.

Next, compressed air was blown onto the flat surface for about 1 second to perform drying, the prepared composition for an adhesive strength test was then applied to the enamel flat surface, and compressed air was blown with a low blowing force. The surface of the applied composition was irradiated with light from a visible light irradiator (Translux 2Wave, manufactured by Heraeus Kulzer GmbH) for 20 seconds. A plastic mold having a diameter of 2.38 mm (manufactured by Ultradent Products, Inc.) was then placed thereon, and a dental composite resin (Venus Diamond, manufactured by Heraeus Kulzer GmbH) was packed, irradiated with light from the visible light irradiator for 20 seconds, and thereby cured. Thereafter, the mold was removed to prepare an adhesive sample for an enamel.

A dentin adherend was prepared using a dentin instead of the enamel. An adhesive sample for a dentin was prepared using the adherend.

### (Measurement Method of Adhesive Strength Test)

The sample was stored in warm water at 37°C for 24 hours, a shear load which was parallel to various adhesive samples and in contact with the surface was then applied at a crosshead speed of 1.0 mm/min using a general-purpose tester (Precise Versatile Material Tester 210X, manufactured by INTESCO Co., Ltd.), and the shear adhesive strength was determined from the shear load at the time when the columnar composition formed on the sample surface was separated from the surface.

### [Method of Bending Strength Test]

A method of a bending strength test in Examples and Comparative Examples of Embodiment A is shown below.

### (Preparation of Test Piece for Bending Strength Test)

A composition for a bending strength test was placed in a stainless steel mold of 2 x 2 x 25 mm, and irradiated with light for 3 minutes on each side, that is, for 6 minutes on both sides using a visible light irradiator (ALPHA LIGHT V manufactured by J. MORITA TOKYO MFG). The test piece taken out from the mold was heat-treated in an oven at 130°C for 2 hours. After cooling the test piece taken out from the oven to room temperature, the test piece was immersed in distilled water in a sealable sample bottle and held at 37°C for 24 hours, and this test piece after immersed was used as the test piece (test piece for a bending strength test).

### (Bending Strength Test)

The test piece prepared by the above method was subjected to a three-point bending test using a tester (Autograph EZ-S manufactured by SHIMADZU CORPORATION) at a distance between fulcrums of 20 mm and a crosshead speed of 1 mm/min, and the elastic modulus and the breaking strength were measured.

### [Method of Water Absorption Test]

A method of a water absorption test in Examples and Comparative Examples of Embodiment A is shown below.

### (Preparation of Test Piece for Water Absorption Test)

A composition for a water absorption test had the same composition as the composition for a bending test, and was prepared according to JIS T 6514:2015 and ISO 4049:2009. For curing, a visible light irradiator (ALPHA LIGHT V manufactured by J. MORITA TOKYO MFG) was used.

### (Water Absorption Test)

The water absorption test was performed according to JIS T 6514:2015 and ISO 4049:2009 using the test piece prepared by the above method (N = 5). Calculation was performed using the measured value of each test piece.

### [Method of Measuring IR Spectrum]

The IR spectrum of the (meth)acrylamide compound obtained in each Production Example was measured using Fourier Transform Infrared Spectroscopy, Spectrum Two/UATR (Universal Attenuated Total Reflectance) manufactured by PerkinElmer Japan Co., Ltd.

The obtained (meth)acrylamide compound was left to stand still at 20°C for 24 hours, and then the infrared absorption spectrum of the (meth)acrylamide compound was measured at 20°C.

### [Production Example 1: Production of NBDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 15.43 parts by mass of NBDA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 21.0 g of methacrylamide (1) as a white solid.

The methacrylamide (1) was subjected to IR spectrum measurement, and the decay of the peak intensity of the amino group at from 3200 cm⁻¹ to 3500 cm⁻¹ was confirmed. The structure of the methacrylamide (1) was confirmed to be the following structure.

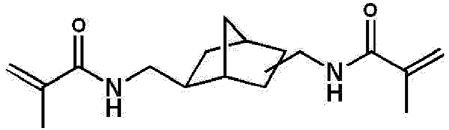

### [Production Example 2: Production of H6XDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 14.23 parts by mass of H6XDAwas added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 5.5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 21.8 g of methacrylamide (2) as a white solid.

The methacrylamide (2) was subjected to IR spectrum measurement, and the decay of the peak intensity of the amino group at from 3200 cm⁻¹ to 3500 cm⁻¹ was confirmed. The structure of the methacrylamide (2) was confirmed to be the following structure.

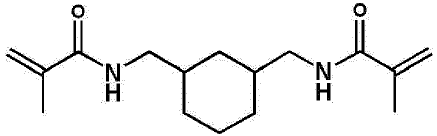

### [Production Example 3: Production of IPDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 17.03 parts by mass of IPDA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6.5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 21.9 g of methacrylamide (3) as a white solid.

The methacrylamide (3) was subjected to IR spectrum measurement, and the decay of the peak intensity of the amino group at from 3200 cm⁻¹ to 3500 cm⁻¹ was confirmed. The structure of the methacrylamide (3) was confirmed to be the following structure.

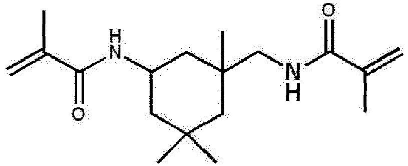

### [Production Example 4: Production of XDA+ Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 13.62 parts by mass of XDAwas added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 21.1 g of methacrylamide (4) as a white solid.

The methacrylamide (4) was subjected to IR spectrum measurement, and the decay of the peak intensity of the amino group at from 3200 cm⁻¹ to 3500 cm⁻¹ was confirmed. The structure of the methacrylamide (4) was confirmed to be the following structure.

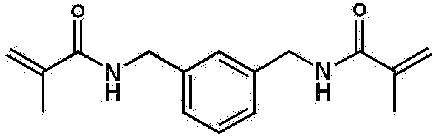

### [Production Example 5: Production of CHDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 11.42 parts by mass of CHDA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6.5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 18.4 g of methacrylamide (5) as a white solid.

The methacrylamide (5) was subjected to IR spectrum measurement, and the decay of the peak intensity of the amino group at from 3200 cm⁻¹ to 3500 cm⁻¹ was confirmed. The structure of the methacrylamide (5) was confirmed to be the following structure.

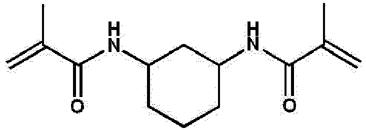

### [Production Example 6: Production of H12MDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 21.03 parts by mass of H12MDAwas added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 7 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 24.8 g of methacrylamide (6) as a white solid.

The methacrylamide (6) was subjected to IR spectrum measurement, and the decay of the peak intensity of the amino group at from 3200 cm⁻¹ to 3500 cm⁻¹ was confirmed. The structure of the methacrylamide (6) was confirmed to be the following structure.

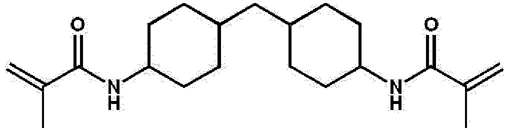

### [Production Example 7: Production of mPDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 10.81 parts by mass of mPDAwas added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 5.5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 19.3 g of methacrylamide (7) as a white solid.

The methacrylamide (7) was subjected to IR spectrum measurement, and the decay of the peak intensity of the amino group at from 3200 cm⁻¹ to 3500 cm⁻¹ was confirmed. The structure of the methacrylamide (7) was confirmed to be the following structure.

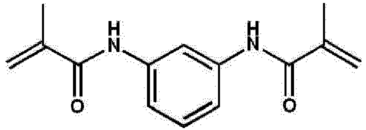

### [Production Example 8: Production of D-400 + Methacrylic Acid Condensate]

Into a thoroughly dried 300 mL four-necked flask equipped with a stirring blade and a thermometer, 8.61 parts by mass of methacrylic acid and 100 parts by mass of DCM were charged and dissolved. Thereafter, the solution was cooled to 10°C, and 19.17 parts by mass of EDC HCL was charged in a divided manner so that the internal temperature did not exceed 10°C. The mixture was dissolved to form a homogeneous solution, and then reacted at 10°C for 0.5 hours. To the solution, 20.00 parts by mass of D-400 was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 18.6 g of methacrylamide (8) as a colorless liquid.

The methacrylamide (8) was subjected to IR spectrum measurement, and the decay of the peak intensity of the amino group at from 3200 cm⁻¹ to 3500 cm⁻¹ was confirmed. The structure of the methacrylamide (8) was confirmed to be the following structure.

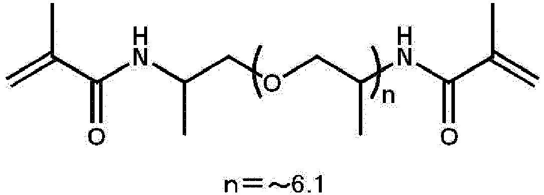

### [Production Example 9: Production of D-2000 + Methacrylic Acid Condensate]

Into a thoroughly dried 300 mL four-necked flask equipped with a stirring blade and a thermometer, 1.72 parts by mass of methacrylic acid and 100 parts by mass of DCM were charged and dissolved. Thereafter, the solution was cooled to 10°C, and 3.83 parts by mass of EDC·HCL was charged in a divided manner so that the internal temperature did not exceed 10°C. The mixture was dissolved to form a homogeneous solution, and then reacted at 10°C for 0.5 hours. To the solution, 20.00 parts by mass of D-2000 was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 5.5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 13.7 g of methacrylamide (9) as a brown liquid.

The methacrylamide (9) was subjected to IR spectrum measurement, and the decay of the peak intensity of the amino group at from 3200 cm⁻¹ to 3500 cm⁻¹ was confirmed. The structure of the methacrylamide (9) was confirmed to be the following structure.

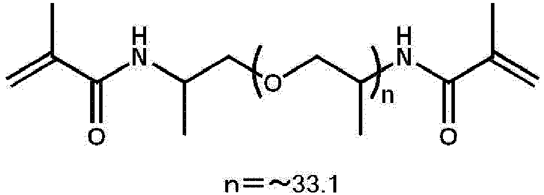

### [Production Example 10: Production of DODA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 14.82 parts by mass of DODA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 17.8 g of methacrylamide (10) as a pale yellow liquid.

The methacrylamide (10) was subjected to IR spectrum measurement, and the decay of the peak intensity of the amino group at from 3200 cm⁻¹ to 3500 cm⁻¹ was confirmed. The structure of the methacrylamide (10) was confirmed to be the following structure.

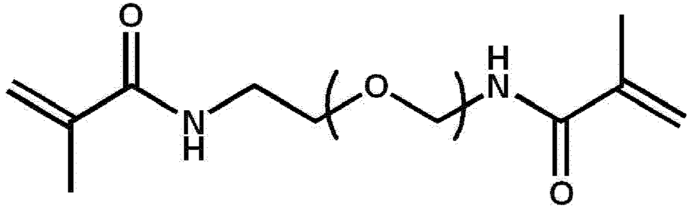

### [Example 1]

In a container, 0.79 parts by mass of the methacrylamide (1) obtained in Production Example 1, 2.38 parts by mass of UDMA, 0.79 parts by mass of 3G, 0.02 parts by mass of CQ, and 0.02 parts by mass of DMBE were placed and stirred at 50°C until the mixture became uniform. Next, 6 parts by mass of silica glass (Fuselex-X (Tatsumori Co., Ltd.) was blended, and the mixture was stirred using a mortar until the mixture became uniform, and then defoamed to prepare a composition for a bending strength test. From the obtained composition for a bending strength test, a water absorption test was performed according to the methods described in the sections of (Preparation of Test Piece for Water Absorption Test) and (Water Absorption Test). The results are shown in Table 1.

### [Examples 2 to 7 and Comparative Examples 1 to 3]

Compositions shown in Table 1 were prepared in the same manner as in Example 1, and a bending strength test and a water absorption test were performed. The results are similarly shown in Table 1.

### [Reference Example 1]

In a container, 3.17 parts by mass of UDMA, 0.79 parts by mass of 3G, 0.02 parts by mass of CQ, and 0.02 parts by mass of DMBE were placed and stirred at 50°C until the mixture became uniform.

Next, 6 parts by mass of silica glass (Fuselex-X (Tatsumori Co., Ltd.) was blended, and the mixture was stirred using a mortar until the mixture became uniform, and then defoamed to prepare a composition for a bending strength test. From the obtained composition for a bending strength test, a water absorption test was performed according to the methods described in the sections of (Preparation of Test Piece for Water Absorption Test) and (Water Absorption Test). The results are shown in Table 1.

**[Table 1]**

| | | Monomer synthesis | | Bending strength | | Water absorbability |
|---|---|---|---|---|---|---|
| | | Production Example | Raw material amine | Elastic modulus [MPa] | Breaking strength [MPa] | Water absorption rate [µg/mm²] |
| Example | 1 | Production Example 1 | NBDA | 5680 | 130 | 7.7 |
| | 2 | Production Example 2 | H6XDA | 5230 | 132 | 8.2 |
| | 3 | Production Example 3 | IPDA | 5630 | 128 | 10.1 |
| | 4 | Production Example 4 | XDA | 5980 | 135 | 7.3 |
| | 5 | Production Example 5 | CHDA | 5620 | 135 | 8.9 |
| | 6 | Production Example 6 | H12MDA | 5580 | 127 | 9.1 |
| | 7 | Production Example 7 | mPDA | 5790 | 132 | 9.4 |
| Comparative Example | 1 | Production Example 8 | D-400 | 4320 | 118 | 18.6 |
| | 2 | Production Example 9 | D-2000 | 4160 | 114 | 22.5 |
| | 3 | Production Example 10 | DODA | 4340 | 105 | 22.9 |
| Reference Example | 1 | UDMA | | 5520 | 136 | 9.6 |

As shown in Table 1, in Examples using the monomer composition for a dental material containing the (meth)acrylamide compound (A) including a cyclic structure and a (meth)acrylamide group, an increase in water absorption rate was suppressed, and the bending strength was favorably maintained.

On the other hand, in Comparative Examples 1 to 3 using the (meth)acrylamide compound not including a cyclic structure, an increase in water absorption rate could not be suppressed. As a result, the bending strength was reduced.

In Examples, in a case in which Reference Example 1 was used as a reference, an increase in water absorption rate was suppressed to the same extent, and the bending strength was favorably maintained.

### [Example 8]

In a container, 0.50 parts by mass of the methacrylamide (1) obtained in Production Example 1, 2.97 parts by mass of UDMA, 0.99 parts by mass of HEMA, 0.50 parts by mass of MDP, 0.02 parts by mass of CQ, and 0.02 parts by mass of DMBE were placed and stirred at 50°C until the mixture became uniform. Next, 5 g of an aqueous solution adjusted to EtOH : DW = 3 : 2 was added, and the mixture was stirred at normal temperature until the mixture became uniform, thereby obtaining a composition for an adhesive strength test. From the obtained composition for an adhesive strength test, an adhesive strength test was performed according to the methods described in the sections of (Preparation of Test Piece for Adhesive Strength Test) and (Bending Adhesive Strength Test). The results are shown in Table 2.

### [Examples 9 to 14 and Comparative Example 4]

Compositions shown in Table 2 were prepared in the same manner as in Example 8, and an adhesive strength test was performed. The results are similarly shown in Table 2.

### [Reference Example 2]

In a container, 3.47 parts by mass of UDMA, 0.99 parts by mass of HEMA, 0.50 parts by mass of MDP, 0.02 parts by mass of CQ, and 0.02 parts by mass of DMBE were placed and stirred at 50°C until the mixture became uniform. Next, 5 g of an aqueous solution adjusted to EtOH : DW = 3 : 2 was added, and the mixture was stirred at normal temperature until the mixture became uniform, thereby obtaining a composition for an adhesive strength test. From the obtained composition for an adhesive strength test, an adhesive strength test was performed according to the methods described in the sections of (Preparation of Test Piece for Adhesive Strength Test) and (Bending Adhesive Strength Test). The results are shown in Table 2.

**[Table 2]**

| | | Monomer synthesis | | Adhesive strength | |
|---|---|---|---|---|---|
| | | Production Example | Raw material amine | Enamel [MPa] | Dentin [MPa] |
| Example | 8 | Production Example 1 | NBDA | 16.4 | 12.3 |
| | 9 | Production Example 2 | H6XDA | 15.8 | 13.2 |
| | 10 | Production Example 3 | IPDA | 13.8 | 10.8 |
| | 11 | Production Example 4 | XDA | 14.1 | 10.2 |
| | 12 | Production Example 5 | CHDA | 14.1 | 10.4 |
| | 13 | Production Example 6 | H12MDA | 12.4 | 9.6 |
| | 14 | Production Example 7 | mPDA | 13.1 | 10.2 |
| Comparative Example | 4 | Production Example 9 | D-2000 | 10.4 | 8.2 |
| Reference Example | 2 | UDMA | | 12.2 | 8.6 |

As shown in Table 2, in Examples using the monomer composition for a dental material containing the (meth)acrylamide compound (A) including a cyclic structure and a (meth)acrylamide group, the adhesive strength to the enamel and the dentin was excellent in a case in which Reference Example 2 was used as a reference.

On the other hand, in Comparative Example 4 using the (meth)acrylamide compound not including a cyclic structure, the adhesive strength to the enamel and the dentin was poor in a case in which Reference Example 2 was used as a reference.

Hereinafter, Embodiment A-1 will be more specifically described with reference to Examples; however, Embodiment A-1 is not limited to the following Examples.

Abbreviations of compounds used in Examples of Embodiment A-1 are shown below.
EDC·HCl: 1-(3-dimethylaminopropyl)-3-ethylcarbodiimide hydrochloride
DCM: dichloromethane
NBDA: bis(aminomethyl)norbornane
H6XDA: 1,3-bis(aminomethyl)cyclohexane
IPDA: isophorone diamine
XDA: m-xylylenediamine
D-400: JEFFAMINE (registered trademark) D-400
D-2000: JEFFAMINE (registered trademark) D-2000
DODA: 1,2-bis(2-aminoethoxy)ethane
UDMA: 2,2,4-trimethylhexamethylene bis(2-carbamoyloxyethyl) dimethacrylate
HEMA: 2-hydroxyethyl methacrylate
MDP: 10-(phosphonooxy)decyl-methacrylate
3G: triethylene glycol dimethacrylate
CQ: camphorquinone
DMBE: 2-butoxyethyl 4-(dimethylamino) benzoate
EtOH: ethanol
DW: distilled water

Structural formulas of NBDA, H6XDA, IPDA, XDA, D-400, D-2000, and DODA are as follows.

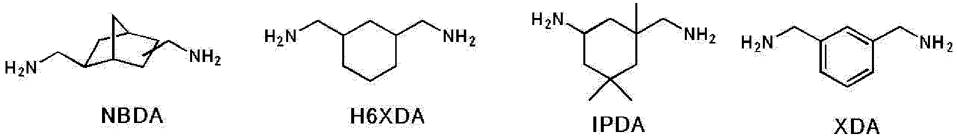

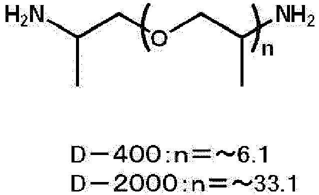

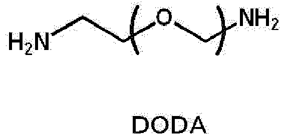

### [Method of Measuring HPLC]

In Examples of Embodiment A-1, the method of measuring HPLC is the same as the method described in [Method of Measuring HPLC] in Examples of Embodiment A.

### [Method of Adhesive Strength Test]

A method of an adhesive strength test in Examples and Comparative Examples of Embodiment A-1 is shown below.

### (Preparation of Test Piece for Adhesive Strength Test)

In Examples of Embodiment A-1, a test piece for an adhesive strength test is produced by the same method as the method described in (Preparation of Test Piece for Adhesive Strength Test) in Examples of Embodiment A.

### (Measurement Method of Adhesive Strength Test)

In Examples of Embodiment A-1, the measurement method of an adhesive strength test is the same as the method described in (Measurement Method of Adhesive Strength Test) in Examples of Embodiment A.

### [Method of Bending Strength Test]

A method of a bending strength test in Examples and Comparative Examples of Embodiment A-1 is shown below.

### (Preparation of Test Piece for Bending Strength Test)

In Examples of Embodiment A-1, a test piece for a bending strength test is produced by the same method as the method described in (Preparation of Test Piece for Bending Strength Test) in Examples of Embodiment A.

### (Bending Strength Test)

In Examples of Embodiment A-1, a bending strength test is performed by the same method as the method described in (Bending Strength Test) in Examples of Embodiment A.

### [Method of Water Absorption Test]

A method of a water absorption test in Examples and Comparative Examples of Embodiment A-1 is shown below.

### (Preparation of Test Piece for Water Absorption Test)

In Examples of Embodiment A-1, a test piece for a water absorption test is produced by the same method as the method described in (Preparation of Test Piece for Water Absorption Test) in Examples of Embodiment A.

### (Water Absorption Test)

In Examples of Embodiment A-1, a water absorption test is performed by the same method as the method described in (Water Absorption Test) in Examples of Embodiment A.

### [Method of Measuring IR Spectrum]

In Examples of Embodiment A-1, a method of measuring an IR spectrum is the same as the method described in [Method of Measuring IR Spectrum] in Examples of Embodiment A.

### [Production Example 1A: Production of NBDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 15.43 parts by mass of NBDA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 21.0 g of methacrylamide (1) as a white solid.

The methacrylamide (1) was subjected to IR spectrum measurement, and the decay of the peak intensity of the amino group at from 3200 cm⁻¹ to 3500 cm⁻¹ was confirmed. The structure of the methacrylamide (1) was confirmed to be the following structure.

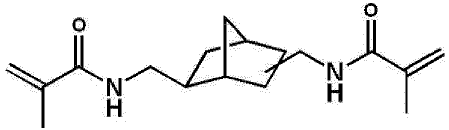

### [Production Example 2A: Production of H6XDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 14.23 parts by mass of H6XDAwas added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 5.5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 21.8 g of methacrylamide (2) as a white solid.

The methacrylamide (2) was subjected to IR spectrum measurement, and the decay of the peak intensity of the amino group at from 3200 cm⁻¹ to 3500 cm⁻¹ was confirmed. The structure of the methacrylamide (2) was confirmed to be the following structure.

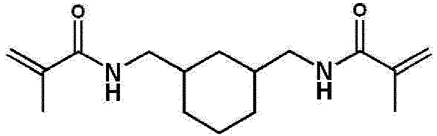

### [Production Example 3A: Production of IPDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 17.03 parts by mass of IPDA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6.5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 21.9 g of methacrylamide (3) as a white solid.

The methacrylamide (3) was subjected to IR spectrum measurement, and the decay of the peak intensity of the amino group at from 3200 cm⁻¹ to 3500 cm⁻¹ was confirmed. The structure of the methacrylamide (3) was confirmed to be the following structure.

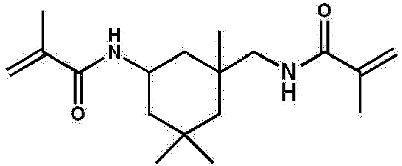

### [Production Example 4A: Production of XDA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 13.62 parts by mass of XDAwas added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 21.1 g of methacrylamide (4) as a white solid.

The methacrylamide (4) was subjected to IR spectrum measurement, and the decay of the peak intensity of the amino group at from 3200 cm⁻¹ to 3500 cm⁻¹ was confirmed. The structure of the methacrylamide (4) was confirmed to be the following structure.

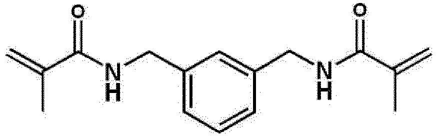

### [Production Example 5A: Production of D-400 + Methacrylic Acid Condensate]

Into a thoroughly dried 300 mL four-necked flask equipped with a stirring blade and a thermometer, 8.61 parts by mass of methacrylic acid and 100 parts by mass of DCM were charged and dissolved. Thereafter, the solution was cooled to 10°C, and 19.17 parts by mass of EDC HCL was charged in a divided manner so that the internal temperature did not exceed 10°C. The mixture was dissolved to form a homogeneous solution, and then reacted at 10°C for 0.5 hours. To the solution, 20.00 parts by mass of D-400 was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 18.6 g of methacrylamide (5) as a colorless liquid.

The methacrylamide (5) was subjected to IR spectrum measurement, and the decay of the peak intensity of the amino group at from 3200 cm⁻¹ to 3500 cm⁻¹ was confirmed. The structure of the methacrylamide (5) was confirmed to be the following structure.

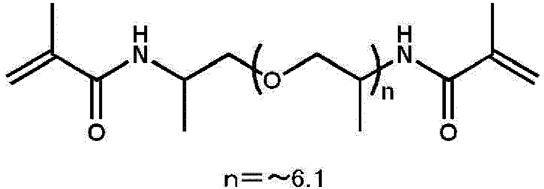

### [Production Example 6A: Production of D-2000 + Methacrylic Acid Condensate]

Into a thoroughly dried 300 mL four-necked flask equipped with a stirring blade and a thermometer, 1.72 parts by mass of methacrylic acid and 100 parts by mass of DCM were charged and dissolved. Thereafter, the solution was cooled to 10°C, and 3.83 parts by mass of EDC HCL was charged in a divided manner so that the internal temperature did not exceed 10°C. The mixture was dissolved to form a homogeneous solution, and then reacted at 10°C for 0.5 hours. To the solution, 20.00 parts by mass of D-2000 was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 5.5 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 13.7 g of methacrylamide (6) as a brown liquid.

The methacrylamide (6) was subjected to IR spectrum measurement, and the decay of the peak intensity of the amino group at from 3200 cm⁻¹ to 3500 cm⁻¹ was confirmed. The structure of the methacrylamide (6) was confirmed to be the following structure.

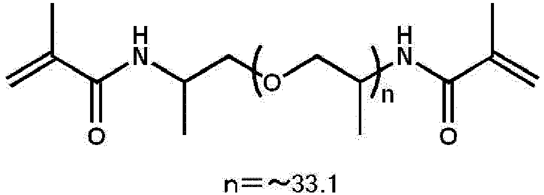

### [Production Example 7A: Production of DODA + Methacrylic Acid Condensate]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 14.82 parts by mass of DODA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6 hours while keeping the reaction temperature at 30°C. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The remaining DCM layer was distilled off with an evaporator to obtain 17.8 g of methacrylamide (7) as a pale yellow liquid.

The methacrylamide (7) was subjected to IR spectrum measurement, and the decay of the peak intensity of the amino group at from 3200 cm⁻¹ to 3500 cm⁻¹ was confirmed. The structure of the methacrylamide (7) was confirmed to be the following structure.

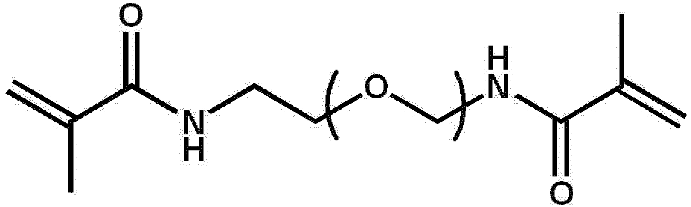

### [Example 1A]

In a container, 0.50 parts by mass of the methacrylamide (1) obtained in Production Example 1A, 2.97 parts by mass of UDMA, 0.99 parts by mass of HEMA, 0.50 parts by mass of MDP, 0.02 parts by mass of CQ, and 0.02 parts by mass of DMBE were placed and stirred at 50°C until the mixture became uniform. Next, 5 g of an aqueous solution adjusted to EtOH : DW = 3 : 2 was added, and the mixture was stirred at normal temperature until the mixture became uniform, thereby obtaining a composition for an adhesive strength test. From the obtained composition for an adhesive strength test, an adhesive strength test was performed according to the methods described in the sections of (Preparation of Test Piece for Adhesive Strength Test) and (Bending Adhesive Strength Test). The results are shown in Table 3.

### [Examples 2A to 4Aand Comparative Example 1A]

Compositions shown in Table 3 were prepared in the same manner as in Example 1A, and an adhesive strength test was performed. The results are similarly shown in Table 3.

### [Reference Example 1A]

In a container, 3.47 parts by mass of UDMA, 0.99 parts by mass of HEMA, 0.50 parts by mass of MDP, 0.02 parts by mass of CQ, and 0.02 parts by mass of DMBE were placed and stirred at 50°C until the mixture became uniform. Next, 5 g of an aqueous solution adjusted to EtOH : DW = 3 : 2 was added, and the mixture was stirred at normal temperature until the mixture became uniform, thereby obtaining a composition for an adhesive strength test. From the obtained composition for an adhesive strength test, an adhesive strength test was performed according to the methods described in the sections of (Preparation of Test Piece for Adhesive Strength Test) and (Bending Adhesive Strength Test). The results are shown in Table 3.

**[Table 3]**

| | | Monomer synthesis | | Adhesive strength | |
|---|---|---|---|---|---|
| | | Production Example | Raw material amine | Enamel [MPa] | Dentin [MPa] |
| Example | 1A | Production Example 1A | NBDA | 16.4 | 12.3 |
| | 2A | Production Example 2A | H6XDA | 15.8 | 13.2 |
| | 3A | Production Example 3A | IPDA | 13.8 | 10.8 |
| | 4A | Production Example 4A | XDA | 14.1 | 10.2 |
| Comparative Example | 1A | Production Example 6A | D-2000 | 10.4 | 8.2 |
| Reference Example | 1A | UDMA | | 12.2 | 8.6 |

As shown in Table 3, Examples 1A and 2A using the (meth)acrylamide compound (A-1) containing two (meth)acrylamide groups and a divalent alicyclic hydrocarbon group, in which a nitrogen atom in each of the two (meth)acrylamide groups isbonded to the divalent alicyclic hydrocarbon group via a methylene group, were superior in adhesive strength to the enamel and the dentin as compared with Examples 3A to 4A and Comparative Example 1A.

### [Example 5A]

In a container, 0.79 parts by mass of the methacrylamide (1) obtained in Production Example 1A, 2.38 parts by mass of UDMA, 0.79 parts by mass of 3G, 0.02 parts by mass of CQ, and 0.02 parts by mass of DMBE were placed and stirred at 50°C until the mixture became uniform. Next, 6 parts by mass of silica glass (Fuselex-X (Tatsumori Co., Ltd.) was blended, and the mixture was stirred using a mortar until the mixture became uniform, and then defoamed to prepare a composition (1) for a bending strength test. From the obtained composition for a bending strength test, a water absorption test was performed according to the methods described in the sections of (Preparation of Test Piece for Water Absorption Test) and (Water Absorption Test). The results are shown in Table 4.

### [Examples 6A to 8A and Comparative Examples 2A to 4A]

Compositions shown in Table 4 were prepared in the same manner as in Example 5A, and a bending strength test and a water absorption test were performed. The results are similarly shown in Table 4.

### [Reference Example 2A]

In a container, 3.17 parts by mass of UDMA, 0.79 parts by mass of 3G, 0.02 parts by mass of CQ, and 0.02 parts by mass of DMBE were placed and stirred at 50°C until the mixture became uniform.

Next, 6 parts by mass of silica glass (Fuselex-X (Tatsumori Co., Ltd.) was blended, and the mixture was stirred using a mortar until the mixture became uniform, and then defoamed to prepare a composition for a bending strength test. From the obtained composition for a bending strength test, a water absorption test was performed according to the methods described in the sections of (Preparation of Test Piece for Water Absorption Test) and (Water Absorption Test). The results are shown in Table 4.

**[Table 4]**

| | | Monomer synthesis | | Bending strength | | Water absorbability |
|---|---|---|---|---|---|---|
| | | Production Example | Raw material amine | Elastic modulus [MPa] | Breaking strength [MPa] | Water absorption rate [µg/mm²] |
| Example | 5A | Production Example 1 | NBDA | 5680 | 130 | 7.7 |
| | 6A | Production Example 2 | H6XDA | 5230 | 132 | 8.2 |
| | 7A | Production Example 3 | IPDA | 5630 | 128 | 10.1 |
| | 8A | Production Example 4 | XDA | 5980 | 135 | 7.3 |
| Comparative Example | 2A | Production Example 5 | D-400 | 4320 | 118 | 18.6 |
| | 3A | Production Example 6 | D-2000 | 4160 | 114 | 22.5 |
| | 4A | Production Example 7 | DODA | 4340 | 105 | 22.9 |
| Reference Example | 2A | UDMA | | 5520 | 136 | 9.6 |

As shown in Table 4, in Examples 5A to 8A, a cured product in which an increase in water absorption rate is suppressed could be obtained.

In particular, as shown in Table 4, in Examples 5A and 6A using the (meth)acrylamide compound (A-1) containing two (meth)acrylamide groups and a divalent alicyclic hydrocarbon group, in which a nitrogen atom in each of the two (meth)acrylamide groups is bonded to the divalent alicyclic hydrocarbon group via a methylene group, an increase in water absorption rate was suppressed and the bending strength was favorably maintained in a case in which Reference Example 2 was used as a reference.

In Examples 5A and 6A, an increase in water absorption rate was suppressed to the same extent or more and the bending strength was favorably maintained as compared with Example 7A using the (meth)acrylamide compound in which one of the nitrogen atoms in the two (meth)acrylamide groups was not bonded to the divalent alicyclic hydrocarbon group via a methylene group, and Example 8A and Comparative Examples 2A to 4A using the (meth)acrylamide compound not including an alicyclic hydrocarbon group.

The entire contents of the disclosures by Japanese Patent Application No. 2021-152359, filed September 17, 2021 and Japanese Patent Application No. 2021-152360, filed September 17, 2021 are incorporated herein by reference.

All the literature, patent application, and technical standards cited herein are also herein incorporated to the same extent as provided for specifically and severally with respect to an individual literature, patent application, and technical standard to the effect that the same should be so incorporated by reference.

## Claims

1. A (meth)acrylamide compound (A), comprising a cyclic structure and a (meth)acrylamide group.

2. The (meth)acrylamide compound (A) according to claim 1, wherein the (meth)acrylamide compound (A) is a reaction product of a (meth)acrylic compound (X) and a primary amine compound (Y) including two amino groups and a cyclic structure, the (meth)acrylic compound (X) being at least one selected from the group consisting of a (meth)acrylic acid and a halide of a (meth)acrylic acid.

3. The (meth)acrylamide compound (A) according to claim 2, wherein the primary amine compound (Y) includes a compound represented by any one of the following Formulae (1-1) to (1-7):

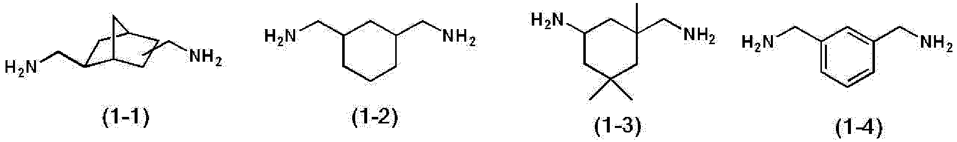

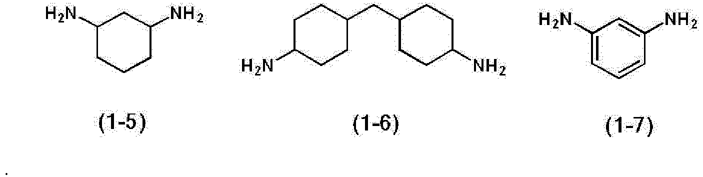

4. The (meth)acrylamide compound (A) according to claim 1, which is a compound represented by the following Formula (1): 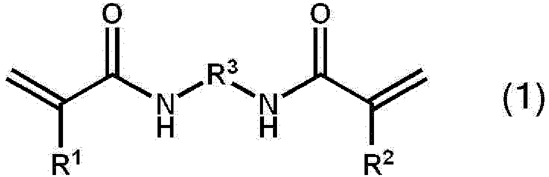 wherein, in Formula (1), each of R¹ and R² independently represents a hydrogen atom or a methyl group, and R³ represents a divalent group including a cyclic structure.

5. The (meth)acrylamide compound (A) according to claim 4, wherein the R³ in Formula (1) is a group represented by the following Formula (1a): 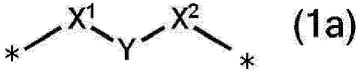 wherein, in Formula (1a), each of X¹ and X² independently represents a single bond or a methylene group, Y represents a divalent linking group having from 6 to 13 carbon atoms including an alicyclic structure or an aromatic structure, and each of two *'s represents a bonding position.

6. The (meth)acrylamide compound (A) according to claim 1, comprising two (meth)acrylamide groups and a divalent alicyclic hydrocarbon group,
wherein a nitrogen atom in each of the two (meth)acrylamide groups is bonded to the divalent alicyclic hydrocarbon group via a methylene group.

7. The (meth)acrylamide compound (A) according to claim 6, which is a reaction product of:
a (meth)acrylic compound (X) which is at least one selected from the group consisting of a (meth)acrylic acid and a halide of a (meth)acrylic acid, and
a primary amine compound (Y1) that includes two amino groups and the divalent alicyclic hydrocarbon group, in which a nitrogen atom in each of the two amino groups is bonded to the divalent alicyclic hydrocarbon group via a methylene group.

8. The (meth)acrylamide compound (A) according to claim 7, wherein the primary amine compound (Y1) includes a compound represented by the following Formula (1-1) or (1-2):

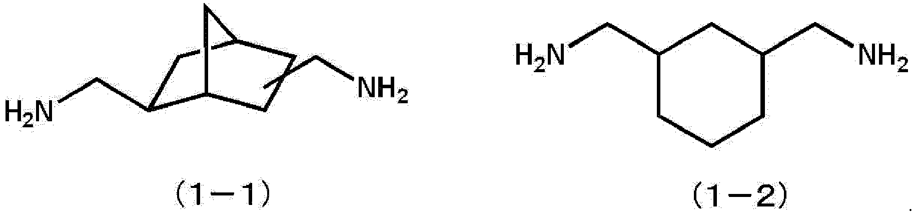

9. The (meth)acrylamide compound (A) according to claim 6, which is a compound represented by the following Formula (1A): 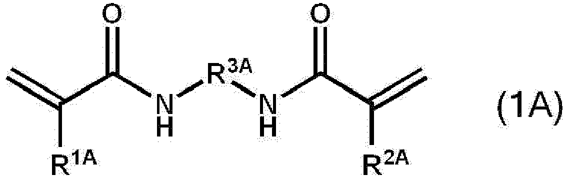 wherein, in Formula (1A), each of R^{1A} and R^{2A} independently represents a hydrogen atom or a methyl group, and R^{3A} represents a divalent group formed by linking a methylene group, a divalent alicyclic hydrocarbon group, and a methylene group in this order.

10. The (meth)acrylamide compound (A) according to claim 9, wherein the R^{3A} in Formula (1A) is a group represented by the following Formula (1Aa): 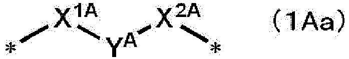 wherein, in Formula (1Aa), each of X^{1A} and X^{2A} independently represents a methylene group, Y^{A} represents a divalent alicyclic hydrocarbon group having from 6 to 9 carbon atoms, and each of two *'s represents a bonding position.

11. The (meth)acrylamide compound (A) according to claim 1, wherein a molecular weight is from 150 to 500.

12. A monomer composition, comprising the (meth)acrylamide compound (A) according to claim 1.

13. The monomer composition according to claim 12, which is used for a dental material.

14. A composition for a dental material, comprising the monomer composition according to claim 12 and a polymerization initiator.

15. A dental material, comprising a cured product of the composition for a dental material according to claim 14.
